**Europäisches Patentamt**

**European Patent Office**

**Office européen des brevets**

(19)

(11) Veröffentlichungsnummer : **0 504 112 A2**

# (12) EUROPÄISCHE PATENTANMELDUNG

(21) Anmeldenummer : **92810171.6**

(22) Anmeldetag : **05.03.92**

(51) Int. Cl.$^5$ : **A61K 9/12**

(30) Priorität : **14.03.91 CH 781/91**

(43) Veröffentlichungstag der Anmeldung :
**16.09.92 Patentblatt 92/38**

(84) Benannte Vertragsstaaten :
**AT BE CH DE DK ES FR GB GR IT LI LU NL PT
SE**

(71) Anmelder : **CIBA-GEIGY AG**
**Klybeckstrasse 141**
**CH-4002 Basel (CH)**

(71) Anmelder : **JAGO PHARMA AG**
**Eptingerstrasse 51**
**CH-4132 Muttenz (CH)**

(72) Erfinder : **Keller, Manfred, Dr.**
**Hegegasse 7**
**W-7812 Bad Krozingen (DE)**
Erfinder : **Herzog, Kurt**
**Hegenheimerstrasse 15**
**CH-4055 Basel (CH)**

(54) **Pharmazeutische Aerosolformulierungen.**

(57)     Die vorliegende Erfindung betrifft eine Aerosolformulierung enthaltend :
    a) ein verflüssigtes Treibgas oder Treibgasgemisch aus der Gruppe der alternativen Treibgase, welche keine abspaltbaren Chloratome besitzen ;
    b) ein nicht-ionisches Tensid aus der Gruppe der monoacetylierten oder diacetylierten Monoglyceride der MYVACET-Reihe ;
    c) einen pharmazeutischen Wirkstoff oder eine Wirkstoffkombination und, falls notwendig,
    d) weitere übliche pharmazeutische Hilfsstoffe, welche sich für Aerosolformulierungen eignen. Die Aerosolformulierung ist insbesondere in der Inhalationstherapie zur Behandlung von Krankheiten der oberen Luftwege wie Asthma oder Rhinitis verwendbar.

EP 0 504 112 A2

Die Erfindung betrifft eine pharmazeutische Zusammensetzung mit chlorfreien Treibgasen zur Anwendung als Aerosolformulierung, Verfahren zur Herstellung dieser pharmazeutischen Zusammensetzung und ihre Anwendung in einem therapeutischen Verfahren, insbesondere in der Inhalationstherapie.

Die Verabreichung von Wirkstoffen mit Aerosolen gewinnt zunehmende Bedeutung, insbesondere mit Inhalationsdosieraerosolen, zur Behandlung von Erkrankungen des Respirationstraktes, z.B. Asthma oder allergischer Rhinitis (Heuschnupfen). Mit einem solchen Aerosol kann der Wirkstoff in wirksamer Dosis an den eigentlichen Anwendungsort, z.B. die Bronchiolen, gelangen, ohne den Gesamtorganismus in dem Umfang zu belasten, wie dies z.B. bei peroraler Verabreichung der Fall ist.

Ein Aerosol ist ein Stoffgemisch, das aus einem gasförmigen Dispersionsmittel und flüssigen oder festen dispersen Bestandteilen besteht (ABC Chemie, Band 1, Seite 23, 2.Auflage 1974, Verlag Harri Deutsch, D-6000 Frankfurt/Main).

Das gasförmige Dispersionsmittel bezeichnet man als Treibgas, das auch aus einer Mischung aus mehreren Treibgasen bestehen kann. Für die pharmazeutische Anwendung bestehen die dispersen Bestandteile aus flüssigen oder festen Wirkstoffen, wobei man zwischen Pulver- oder Suspensionsaerosolen (fest/gasförmig) und Emulsions- oder Flüssigaerosolen (flüssig/gasförmig) unterscheidet. Aerosole können ausserdem weitere Hilfsstoffe wie Tenside enthalten.

Als Aerosole werden insbesondere Stoffgemische in druckdichten Behältnissen (Sprühdosen) bezeichnet, welche das gasförmige Dispersionsmittel bei Raumtemperatur in flüssigem Zustand unter Überdruck enthalten. Die druckdichten Behältnisse sind mit einem Dosierventil verschlossen. Bei der Verabreichung entweicht nach Betätigen des Dosierventils aus dem unter Überdruck stehenden Behältnis das eigentliche Aerosol mit dem dispergierten Wirkstoff, z.B. im Treibgas dispergierten festen Teilchen oder Tröpfchen.

Diskussionen und Untersuchungen über die Ursache der Schädigung der atmosphärischen Ozonschicht durch Fluorchlorkohlenwasserstoffe (FCKW) haben dazu geführt, dass deren technische Verwendung als Treibgase in Sprühdosen in vielen Ländern vom Gesetzgeber reglementiert wurde. Aus verschiedenen Forschungsarbeiten ist bekannt, dass nur die Chloratome in den FCKW und die daraus entstehenden reaktionsfähigen Radikale für die Schädigung der Ozonschicht verantwortlich sind.

Es werden daher für technische, nicht pharmazeutische Anwendungen zunehmend sogenannte alternative Treibgase oder Treibmittel, z.B. gesättigte Kohlenwasserstoffe wie Propan oder n-Butan, oder chlorfreie Fluorkohlenwasserstoffe (HFKW oder FKW), z.B. Tetrafluorethan, oder Mischungen dieser Treibgase verwendet. Allein vom ökologischen Aspekt drängt es sich auf, auch für pharmazeutische Anwendungen potentiell Ozonschicht-schädigende FCKW durch umweltverträglichere, alternative Treibgase zu ersetzen.

Für pharmazeutische Anwendungen bzw. als Treibgase oder Treibmittel für pharmazeutische Aerosole eignen sich solche Treibgase, welche sich unter Druck bei Raumtemperatur veflüssigen lassen und bei Inhalationen oder topischer Anwendung toxikologisch unbedenklich sind und keine Nebenwirkungen besitzen.

Besonders für die Inhalationstherapie besteht ein Bedarf an Aerosolen, worin die im Treibgas dispergierten festen Teilchen oder Tröpfchen einen bevorzugten Durchmesser von ca. 0,5 - 6 μm besitzen. Suspensionen sollten den Wirkstoff möglichst homogen verteilt enthalten, so dass dieser nach dem Aufschütteln möglichst lange im fein dispersen Zustand bleibt. Damit sich die Grössenverteilung der Partikel nicht verändert, sollte bei Suspensionsaerosolen der Wirkstoff nicht im Treibgas/Hilfsstoffgemisch gelöst sein.

In der Europäischen Patentanmeldung 372 777 sind Aerosolformulierungen mit dem "alternativen" Treibmittel Tetrafluorethan (134a) beschrieben. Zur Erhöhung der Stabilität von Suspensionen in diesem verflüssigten Treibmittel wird vorgeschlagen, ein Suspendierhilfsmittel in Form eines nicht-ionischen Tensids, z.B. Sorbitanfettsäureester vom Typ SPAN, z.B. SPAN 85 (Sorbitantrioleat), zu verwenden. Aufgrund unzureichender Löslichkeit solcher Tenside im verflüssigten Treibmittel 134a wird ein Lösungsmittel, z.B. Ethanol, hinzubesetzt, welches eine höhere Polarität als das Treibmittel selbst besitzt.

Der vorliegenden Erfindung liegt die Aufgabe zugrunde, ein spezielles Suspendierhilfsmittel für Wirkstoffe in Aerosolformulierungen zu finden, das sich in verflüssigten "alternativen" Treibgasen besser löst als die bisher dafür verwendeten bekannten Suspendierhilfsmittel. Bei der Lösung dieser Aufgabenstellung wurde überraschenderweise gefunden, dass nicht-ionische Tenside aus der Gruppe der monoacetylierten oder diacetylierten Monoglyceride in den genannten "alternativen" Treibgasen, insbesondere in Heptafluorpropan (227), gut löslich sind und die Herstellung von homogenen Suspensionen begünstigen, wobei sie ausserdem hervorragende Schmierungseigenschaften für das Dosierventil aufweisen.

Die vorliegende Erfindung betrifft eine pharmazeutische Zusammensetzung zur Anwendung als Aerosol enthaltend:

a) ein veflüssigtes Treibgas oder Treibgasgemisch mit einem Dampfdruck von mehr als 1bar und weniger als 6 bar (20°C) aus der Gruppe der unsubstituierten oder partiell bis vollständig fluorierten Kohlenwasserstoffe;

b) ein nicht-ionisches Tensid aus der Gruppe der monoacetylierten oder diacetylierten Monoglyceride;

c) einen pharmazeutischen Wirkstoff oder eine Wirkstoffkombination und gegebenenfalls

d) weitere übliche pharmazeutische Hilfsstoffe, welche sich für Aerosolformulierungen eignen.

In einer besonders bevorzugten Ausführungsform enthält das Aerosol das alternative Treibgas Heptafluorpropan (227). Dieses Treibgas hat den Vorteil des niedrigeren Dampfdruck von ca. 4 bar verglichen mit dem höheren Dampfdruck von ca. 5,7 bar bei derselben Temperatur, welche das alternative Treibgas Tetrafluorethan (134a) aufweist.

Der Begriff pharmazeutische Zusammensetzung definiert ein Stoffgemisch, das sich für verschiedene Anwendungen als Aerosol, vorzugsweise für Inhalationen, aber auch topisch, an Mensch und Tier, vorzugsweise am Menschen eignet und zur Behandlung von diversen Krankheiten, z.B. Asthma oder allergischer Rhinitis, verwendbar ist.

Der Begriff Aerosol wird bereits weiter vorn definiert. In dem Druckgefäss zur Erzeugung des Aerosols selbst kann der Wirkstoff oder die Wirkstoffkombination entweder in fester Form als Suspension oder in flüssiger Form als Emulsion oder Lösung in dem zu einer Flüssigkeit komprimierten Treibgas oder Treibgasgemisch enthalten sein. In der Fachliteratur wird gelegentlich das Druckgefäss mit Inhalt und Dosierventil als Dosieraerosol bezeichnet. Im Rahmen der Beschreibung dieser Erfindung soll der Begriff Dosieraerosol nur den Inhalt der Druckgefässe bezeichnen.

Das Treibgas oder Treibgasgemisch der Komponente a) wird so gewählt, dass es bei einer Temperatur von ca. 20°C in einem flüssigen Aggregatzustand vorliegt und einen Mindestüberdruck höher als ca. 1 bar bis zu einem maximalen Überdruck von ca. 6 bar aufweist. Geeignet sind daher Treibgase oder Treibgasgemische, welche einen konstanten Innendruck im Dosiergefäss bis zu dessen vollständiger Entleerung aufweisen und aufgrund der weiter vorn erwähnten Umweltproblematik keine abspaltbaren Chloratome besitzen.

Solche Treibgase oder Treibgasgemische sind an sich für die Herstellung von pharmazeutischen Aerosolen bekannt, z.B. unsubstituierte gesättigte Kohlenwasserstoffe, z.B. n-Propan, n-Butan oder Isobutan oder Gemische davon, oder partiell fluorierte oder vollständig fluorierte (perfluorierte) Kohlenwasserstoffe.

Partiell fluorierte Kohlenwasserstoffe leiten sich von aliphatischen Kohlenwasserstoffen mit vorzugsweise 1-4 C-Atomen, z.B. Methan, Ethan, Propan, n-Butan oder Isobutan, oder cycloaliphatischen Kohlenwasserstoffen mit vorzugsweise 3 und 4 C-Atomen, ab, z.B. Cyclopropan oder Cyclobutan, indem die Wasserstoffatome durch mindestens ein Fluoratom und vorzugsweise mindestens zwei Fluoratome so substituiert sind, dass mindestens ein Wasserstoffatom und damit eine Kohlenwasserstoffbindung im Molekül bestehen bleibt.

Vollständig fluorierte (perfluorierte) Kohlenwasserstoffe leiten sich von den genannten aliphatischen Kohlenwasserstoffen mit 1-4 C-Atomen und den genannten cycloaliphatischen Kohlenwasserstoffen mit 3 und 4 C-Atomen durch Substitution der Wasserstoffatome durch entsprechende Fluoratome ab.

Geeignete partiell oder vollständig fluorierte Kohlenwasserstoffe sind z.B. Methanderivate mit 1-4, Ethanderivate mit 1-6, Propanderivate mit 1-8, n-Butanderivate mit 1-10, Cyclopropanderivate mit 1-6 und Cyclobutanderivate mit 1-8 Fluoratomen. In diesen partiell oder vollständig fluorierten Kohlenwasserstoffen kann (können) das (die) Wasserstoffatom(e) sich an verschiedenen Stellen des Kohlenwasserstoffgerüstes befinden. Für partiell fluorierte Kohlenwasserstoffe sind folgende Isomeriefälle möglich:

Ist nur ein Wasserstoffatom vorhanden, kann dieses in den Propan- und Butanderivaten endständig sein oder sich an einem Zwischenglied der C-Kette befinden.

Bei mehr als einem Wasserstoffatom sind für die Ethan-, Propan-, n-Butan-, Cyclopropanund Cyclobutanderivate sowie für Kohlenwasserstoffe mit einer noch höheren Anzahl an C-Atomen noch weitere Isomeriefälle möglich. Die Wasserstoffatome könne teilweise oder vollständig endständig sein und sich teilweise oder ganz an einem oder an verschiedenen Zwischengliedern der C-Ketten befinden. "Gemischte" Isomeriefälle sind auch möglich, indem die Wasserstoffatome sich in unterschiedlicher Verteilung bei aliphatischen Derivaten am endständigen C-Atom und an denselben oder an verschiedenen Kohlenstoff-Zwischengliedern oder bei cycloaliphatischen Derivaten sich an denselben oder an verschiedenen Kohlenstoffringgliedern befinden.

Zur Abkürzung der üblichen Nomenklatur und zur Unterscheidung der genannten partiell fluorierten Kohlenwasserstoffe sowie auch der im folgenden genannten vollständig fluorierten Kohlenwasserstoffe sind Codebezeichnungen geläufig, die in Pharmazeutische Technologie, H.Sucker, P.Fuchs, P.Speiser (Herausgeber), Thieme Verlag, D-7000 Stuttgart 1978, auf der Seite 735 erläutert und ebenfalls auf FCKW anwendbar sind. Für die genannten zahlreichen Isomeriefälle sind Zusatzbezeichnungen mit den Buchstaben a,b... üblich.

Als partiell fluorierte Kohlenwasserstoffe sind Tetrafluorethan ( 134 und 134a), Trifluorethan (143a), Difluorethan (152 und 152a) und Heptafluorpropan (227) bevorzugt.

Ein nicht-ionisches Tensid aus der Gruppe der monoacetylierten oder diacetylierten Monoglyceride ist ein Monoglycerid (Glycerin mit einer gesättigten oder ungesättigten Fettsäure verestert), das zusätzlich zum Acylrest einer Fettsäure noch vorzugsweise einen oder auch zwei Acetylreste aufweist. Der Acylrest leitet sich vorzugsweise von einer ungesättigten Fettsäure mit mehr als zehn und einer geraden Anzahl an C-Atomen ab.

Bevorzugt ist ein Monoglycerid, welches aus einem Gemisch von monoacetylierten oder diacetylierten Monoglyceriden unter Anwendung der üblichen Trennmethoden, z.B. fraktionierte Destillation, erhältlich ist.

Das acetylierte Monoglycerid enthält als Acylrest einer gesättigten Fettsäure z.B. einen $C_{10-20}$Alkanoylrest mit gerader Anzahl an C-Atomen, z.B. n-Dodecanoyl, n-Tetradecanoyl, n-Hexadecanoyl, n-Octadecanoyl oder n-Icosanoyl.

Das acetylierte Monoglycerid enthält als Acylrest einer ungesättigten Fettsäure vorzugsweise einen $C_{10-20}$Alkenoylrest mit gerader Anzahl an C-Atomen, z.B. 9-cis-Dodecenoyl, -Tetradecenoyl oder -Hexadecenoyl, 6-cis- oder 6-trans-Octadecenoyl, 9-cis- oder 9-trans-Octadecenoyl oder 11-cis-Octadecenoyl.

Besonders bevorzugt sind acetylierte Monoglyceride, welche kommerziell unter dem Warenzeichen MYVACET (Eastman) erhältlich und als Zusatzsstoffe für verarbeitete Lebensmittel von Gesundheitsbehörden, z.B. der FDA in den USA, zugelassen worden sind. Acetylierte Monoglyceride der MYVACET-Reihe finden als Schmiermittel, Weichmacher, nicht-ionische Emulgatoren und Lösungsvermittler Verwendung. Besonders bevorzugt sind die kommerziell unter der Bezeichnung MYVACET 5-07, 7-00, 7-07, 9-08, 9-40 und 9-45 erhältlichen Produkte.

Besonders geeignet sind vor allem die flüssigen acetylierten Monoglyceride, z.B. MYVACET 9-40 und MYVACET 9-45 K, die sich wie folgt charakterisieren lassen:

Erstarrungspunkt:      7-8°C  
Dichte bei 20°C:      0,98-0,99 [g/cm³]  
Hydroxylzahl:      15  
Säurezahl:      3  
Jodzahl:      40-51  
Verseifungszahl:      370-380

Überraschenderweise wurde nun gefunden, dass sich unter Verwendung von flüssigen MYVACET-Typen, insbesondere mit Wirkstoffen aus der Gruppe der Betasympathomimetika, z.B. Salbutamol oder Formoterolfumarat, in den genannten alternativen, nicht die Ozonschicht schädigenden verflüssigten Treibgasen oder Treibgasmischungen der Komponente a) besonders homogene und stabile Suspensionen herstellen lassen. Überraschenderweise wurde ebenfalls gefunden, dass sich durch Zugabe eines polaren Lösungsmittels wie Ethanol unerwünschte Adhäsionen an der Wandung des Druckbehälters vermeiden lassen. Ausserdem wurde gefunden, dass mit der Kombination MYVACET und Ethanol auch andere Wirkstoffe aus chemisch unterschiedlichen Stoffklassen mit Treibgasen wie 227 oder 134a und n-Butan in homogene und leicht redispergierbare Suspensionen überführt werden können, welche als Aerosolformulierungen anwendbar sind.

Das nicht-ionische Tensid b) ist in der pharmazeutischen Zusammensetzung in einer Konzentration von ca. 0,0001 bis 5,0, vorzugsweise ca. 0,001 bis 0,5 Gew.-%, enthalten.

Für die Aerosolformulierung eignen sich im Prinzip alle Wirkstoffe oder Wirktoffkombinationen c), welche gegenüber der Treibgaskomponente a) und der Komponente b), nicht-ionisches Tensid, sowie vorhandenen zusätzlichen Hilfsstoffen inert sind. Die Auswahl der Wirkstoffe wird durch die Indikationsstellung definiert und durch die Aufgabenstellung begrenzt, den gewählten Wirkstoff bzw. die Wirkstoffkombination als Aersolformulierung, vorzugsweise per Inhalation, aber auch topisch zu applizieren.

Geeignete Wirkstoff sind beispielsweise:

Alkaloide: Bromocriptin, Ergotamin, Atropin;

Antiallergika: Azelastin, Cetirizin, Epinastin, Nedocromil, Natriumcromoglicat, Ketotifen, Pemirolast, Traxanost, Amlexanox, Protopin, Tepoxalin, Midaglizol, Picumast, Quazolast, Repirinast, Suplatast;

Antibiotika: Fusefungin, Gentamycin, Neomycin, Minocyclin, Erythromycin, Streptomycin, Ofloxacin, Ciprofloxacin, Cephalexin, Cefatrizin, Cefaclor, Ceftriaxon;

Antimykotika: Clotrimazol, Ketoconazol, Amorolfin, Bifonazol;

Betasympathomimetika: Bambuterol, Salbutamol, Formoterol, Salmeterol, Pirbuterol, Carbuterol, Clenbuterol, Reproterol, Rimiterol, Hexprenalin, Fenoterol, Bitolterol, Terbutalin, Mabuterol, Tulobuterol;

Anticholinergika: Ipratropiumbromid, Oxitropiumbromid, Telenzepin, Troventol;

Kaliumkanalöffner: Cromakalim,, Lemakalim;

Kortikoide: Budesonid, Flunisolid, Beclometason, Fluticason, Triamcinolon, Fluocortinbutyl,Butixocort, Cloprednol, Fluticason, Mometason, Tipredan;

Mukolytika: Acetylcystein, Ambroxol, Carbocystein, Furosemid, Amilorid;

Phosphodiesterase-Inhibitoren: Theophyllin, Isbuphyllin, Zandaverin, Enprophyllin;

Thromboxaninhibitoren: Vapiprost, Ozagrel;

Leukotrieninhibitoren: Bunaprolast, Ibudilast;

Plättchenaggregationsfaktor(PAF)-Antagonisten, z.B. Ginkgolide oder Mequitamium;

Antiinfektika: Pentamidin, Hydroxychloroquin;

Cytostatika: Methotrexat.

Besonders bevorzugt sind solche Wirkstoffe, welche in der Inhalationstherapie zur prophylaktischen und/oder akuten Behandlung von Bronchialobstruktionen wie Asthma Verwendung finden, z.B. Salbutamol, Formoterol, Salmeterol, Dinatriumcromoglicat, Nedocromil, Oxitropium, Ipratropium, Budesonid, Beclometason, Flunisolid und Triamcinolon.

Die genannten Wirkstoffe können, falls sie eine salzbildende Gruppe aufweisen, als freie Verbindungen oder in Form ihrer pharmazeutisch annehmbaren Salze im Aerosol vorhanden sein.

Das Aerosol enthält einen Mengenanteil von ca. 0,0001-5,0 Gew.-% Wirkstoff oder Wirkstoffkombination, vorzugsweise ca. 0,001 -2,5 Gew.-%.

Der pharmazeutischen Zusammensetzung können beispielsweise übliche pharmazeutische Hilfsstoffe beigemischt sein, welche sich für Aerosolformulierungen eignen. So kann man beispielsweise ein Lösungsmittel hinzusetzen, welches eine höhere Polarität als die verflüssigte Treibgaskomponente a) aufweist. Da die Treibgase oder Treibgasgemische der Komponente a) wenig polar sind, eignen sich als Zusatz zahlreiche andere polarere Lösungsmittel wie Ethanol, Isopropanol, Propylenglycol, Dimethylether sowie Mischungen dieser Lösungsmittel, die in beliebigen Konzentrationen, z.B. von ca. 0,1 bis 30 Gew.-%, hinzugesetzt werden können.

Als zusätzliche Hilfsstoffe können zusätzlich zur Komponente b) noch weitere nicht-ionische Tenside hinzugesetzt werden, welche die Benetzung und Dispersionsfähigkeit von pharmazeutischen Wirkstoffen noch weiter erhöhen und/oder als Schmiermittel die mechanischen Ventilfunktionen verbessern sowie die Ablagerung von Feststoffen durch Absorption an der Innenseite des Druckbehälters vermeiden.

Solche zusätzlichen nicht-ionischen Tenside sind z.B. Sorbitanfettsäureester, z.B. Sorbitantrioleat, -sesquioleat, -monooleat, oder -monolaurat, welche z.B. unter der Bezeichnung SPAN kommerziell erhältlich sind, z.B. SPAN 85, 80 und 20, Polyoxyethylensorbitanester, z.B. Polyoxyethylen(20)sorbitanmonolaurat oder -monooleat, z.B. Ester, welche unter der Bezeichnung TWEEN kommerziell erhältlich sind, z.B. TWEEN 20, 40, 60 und 80, Oleyl-, Stearyl- oder Laurylpolyoxyethylenester, welche unter der Bezeichnung BRIJ oder GENAPOL kommerziell erhältlich sind, z.B. BRIJ 92, 72, 30 oder GENAPOL 0-020, sowie Blockcopolymere, welche unter der Bezeichnung SYNPERONIC kommerziell erhältlich sind.

Weitere Hilfsstoffe sind z.B. pharmazeutisch zugelassene Öle, z.B. Öle pflanzlicher Herkunft, z.B. aus Mais, Oliven, Baumwollsamen, Raps oder Sonnenblumen, Phospholipide, z.B. synthetisches Lecithin oder natürliche Lecithinderivate, welche unter der Bezeichnung EPIKURON kommerziell erhältlich sind, Diethylenglycoldioleat, Tetrahydrofurfuryloleat, Ethyloleat, Isopropylmyristat, Glyceryltrioleat, Glycerylmonolaurat, -oleat oder -ricinoleat, Cetylalkohol, Polyethylenglycol 400, Polyolfettsäureester oder Cetylpyridiniumchlorid. Es können noch Geschmackskorrigentien wie Saccharin, Aspartame sowie Aromen, z.B. Dentomint, hinzugesetzt werden.

Die genannten Hilfsstoffe der Komponente d) können in einem Mengenverhältnis von ca. 0,0001 bis 10 Gew.-%, vorzugsweise ca. 0,001 bis 1 Gew.-%, bezogen auf das Gesamtgewicht der pharmazeutischen Formulierung, hinzugesetzt werden.

Die vorliegende Erfindung betrifft vorzugsweise eine pharmazeutische Zusammensetzung enthaltend:
a) ein Treibgas oder Treibgasgemisch aus der Gruppe Propan, n-Butan, Isobutan, Di-, Tri- oder Tetrafluorethan (134a) und Heptafluorpropan (227);
b) ein nicht-ionisches Tensid aus der Gruppe der monoacetylierten Monoglyceride;
c) einen pharmazeutischen Wirkstoff oder eine Wirkstoffkombination aus der Gruppe der Antiallergika, z.B. Dinatriumcromoglicat oder Nedocromil, Betasympathomimetika, z.B. Salbutamol, Salmeterol oder Formoterol, Anticholinergika, z.B. Oxitropium- oder Ipratropiumbromid, und Kortikoide, z.B. Budesonid, Flunisolid, Beclometason oder Triamcinolon,und gegebenenfalls
d) weitere übliche pharmazeutische Hilfsstoffe, welche sich für Aerosolformulierungen eignen.

Die vorliegende Erfindung betrifft vorzugsweise eine pharmazeutische Zusammensetzung enthaltend:
a) ein Treibgas oder Treibgasgemisch aus der Gruppe Propan, n-Butan, Isobutan, Tetrafluorethan (134a) und Heptafluorpropan (227);
b) ein nicht-ionisches Tensid aus der Gruppe der monoacetylierten Monoglyceride;
c) einen pharmazeutischen Wirkstoff oder eine Wirkstoffkombination aus der Gruppe Dinatriumcromoglicat, Salbutamol, Salmeterol, Formoterol, Oxitropiumbromid, Ipratropiumbromid, Budesonid, Flunisolid, Beclometason und Triamcinolon und gegebenenfalls
d) weitere übliche pharmazeutische Hilfsstoffe, welche sich für Aerosolformulierungen eignen.

Die vorliegende Erfindung betrifft in erster Linie eine pharmazeutische Zusammensetzung enthaltend:
a) das Treibgas Heptafluorpropan (227);
b) ein nicht-ionisches Tensid aus der Gruppe der monoacetylierten Monoglyceride;
c) einen pharmazeutischen Wirkstoff oder eine Wirkstoffkombination aus der Gruppe Dinatriumcromoglicat, Salbutamol, Salmeterol, Formoterol, Oxitropiumbromid, Ipratropiumbromid, Budesonid, Flunisolid, Beclometason und Triamcinolon und gegebenenfalls

d) weitere übliche pharmazeutische Hilfsstoffe, welche sich für Aerosolformulierungen eignen.

Ebenfalls Gegenstand der vorliegenden Erfindung ist ein Herstellungsverfahren für die pharmazeutische Zusammensetzung, welche auf an sich bekannte Weise erfolgt und beispielsweise in der weiter vorn erwähnten Pharmazeutischen Technologie auf den Seiten 736-737 beschrieben ist.

Die pharmazeutische Zusammensetzung lässt sich herstellen, indem man in einem druckdichten Behältnis den pharmazeutischen Wirkstoff oder die Wirkstoffkombination c) vorlegt und in beliebiger Reihenfolge der Massnahmen das nicht-ionische Tensid b), gegebenenfalls die üblichen pharmazeutischen Hilfsstoffe d), und dazu das Treibgas oder Treibgasgemisch a) einleitet, homogenisiert und die homogene Mischung in für Aerosolformulierungen geeignete Dosierbehälter abfüllt.

Liegt der Wirkstoff oder liegen die Wirkstoffe in fester, z.B. kristalliner, Form vor, sind diese zu zerkleinern, vorzugsweise zu mmikronisieren. Als Obergrenze der durchschnittlichen Teilchengrösse wird bei topischen Formulierungen ein durchschnittlicher Teilchendurchmesser von weniger als 100, bei Inhalationsformulierungen zwecks Deposition im Respirationstrakt von weniger als 10 Mikrometern angesehen. Eine Teilchengrösse von ca. 0,1-5 Mikrometern ist bei Inhalationsformulierungen bevorzugt, welche durch Anwendung von üblichen Zerkleinerungsverfahren erzielt werden kann, z.B. Mahlen in einer Luftstrahlmühle.

In einem offenen Ansatz-Druckbehälter, welcher mit einer Rühr- und Homogenisiervorrichtung versehen ist, wird die Komponente c) eingewogen und nach Massgabe der Formulierungsvorschrift in der jeweils geeigneten Reihenfolge mit den Komponenten b) - Tensid - und gegebenenfalls d) übrige Hilfsstoffe, z.B. Ethanol, gefüllt. Man verschliesst das Druckgefäss und leitet das Treibgas oder Treibgasgemisch der Komponente a) ein. Der Wirkstoff wird in der Treibgas-Hilfsstoffmischung in der üblichen Weise, z.B. durch Rühren, Schütteln oder Behandeln mit Ultraschall, homogenisiert. Mit den bekannten Abfülltechniken wird der Inhalt des Druckbehälters durch das Ventil in Dosierbehälter gefüllt, z.B. druckdichte Behälter aus Weissblech oder Aluminium, welche vulgär als Sprüh- oder Spraydosen bezeichnet werden und mit den üblichen Dosierventilen versehen sind. Die druckfesten Ventile geben bei Inhalations-Dosieraerosolen ca. 25- 100 Mikroliter ab.

Ebenfalls Gegenstand der Erfindung ist die Anwendung der pharmazeutischen Zusammensetzung in therapeutischen Verfahren, für die aufgrund der Indikationsstellung der darin enthaltenen Wirkstoffe oder Wirkstoffkombinationen ein Bedürfnis besteht. Insbesondere wird die pharmazeutische Zusammensetzung für die Inhalationsbehandlung allergischer Erkrankungen der Luftwege wie Asthma oder allergischer Rhinitis (Heuschnupfen) angewendet, insbesondere wenn das Aerosol Wirkstoffe wie Formoterol, Dinatriumcromoglycat oder Salbutamol enthält. Die Behandlung erfolgt vorzugsweise durch orale oder nasale Inhalation beim Menschen.

Ebenfalls Gegenstand der Erfindung ist die Verwendung eines nicht-ionischen Tensids aus der Gruppe der monoacetylierten oder diacetylierten Monoglyceride zur Herstellung einer pharmazeutischen Zubereitung, welche als Aerosol anwendbar ist. Besonders bevorzugt ist die Verwendung eines flüssigen, nicht-ionischen acetylierten Monoglycerides, welches unter der Bezeichnung MYVACET bekannt ist.

Die folgenden Beispiele illustrieren die Erfindung:

In einem vorgetrockneten Ansatz-Druckbehälter wird der auf eine Teilchengrösse von weniger als 6 Mikrometern mikronisierte Wirkstoff gemäss Rezeptur eingewogen und mit dem Treibgas oder Treibgasgemisch versetzt, welches den Hilfsstoff Ethanol, das nicht-ionische Tensid MYVACET, sowie gegebenenfalls weitere Hilfsstoffe in der jeweiligen Konzentration gemäss Rezepturbeispiel enthält. Nach Rühren und Homogenisieren wird die unter Druck stehende Suspension gegebenenfalls mit Treibgas verdünnt und mit der üblichen Druckfülltechnik in mit einem Dosierventil verschlossene Aluminium- oder Glasbehälter mit einem Volumen von ca. 20 ml abgefüllt. In den Rezepturbeispielen ist die Einwaage jeweils in Gewichtsprozenten angegeben:

| Rezepturbeispiel Nr. | 1 | 2 | 3 | 4 | 5 | 6 | 7 |
|---|---|---|---|---|---|---|---|
| Formoterol-fumarat | 0,017 | 0,017 | 0,017 | 0,017 | 0,017 | 0,017 | 0,017 |
| Ipratropiumbromid | | | 0,086 | 0,086 | 0,086 | | |
| MYVACET 9-40 | 0,030 | 0,030 | 0,043 | 0,086 | 0,0152 | | |
| MYVACET 9-45 | | | | | | 0,017 | 0,034 |
| SPAN 85 | | | | | | 0,019 | |
| Ethanol | | | | | | 5,0 | 5,0 |
| n-Butan | | 69,953 | 69,946 | | 69,745 | | |
| Tetrafluorethan | | 30,0 | 30,0 | | 30,0 | | |
| Heptafluorpropan | 99,953 | | | 99,810 | | 94,947 | 94,949 |

| Rezepturbeispiel Nr. | 8 | 9 | 10 | 11 | 12 |
|---|---|---|---|---|---|
| Formoterol-fumarat | 0,017 | 0,017 | 0,03 | 0,017 | 0,03 |
| Ipratropiumbromid | | | | 0,086 | 0,154 |
| MYVACET 9-40 | | | 0,03 | | |
| MYVACET 9-45 | 0,008 | 0,034 | | 0,017 | 0,153 |
| SPAN 85 | | | | | |
| Ethanol | 2,6 | | | | |
| n-Butan | | | 69,0 | | |
| Tetrafluorethan | | 99,949 | 30,94 | | 69,7 |
| Heptafluorpropan | 97,375 | | | 99,88 | 29,963 |

EP 0 504 112 A2

| Rezepturbeispiel Nr. | 13 | 14 | 15 | 16 | 17 |
|---|---|---|---|---|---|
| Salbutamol-Base | 0,143 | 0,143 | 0,143 | | |
| Dinatriumcromoglicat | | | | 1,5 | 1,5 |
| Budesonid | | | | | |
| MYVACET 9-40 | 0,014 | 0,014 | | 0,15 | 0,1 |
| MYVACET 9-45 | | | 0,14 | | |
| Ethanol | 5,0 | 5,0 | 1,0 | 5,0 | 5,0 |
| n-Butan | | 64,843 | | | 65,1 |
| Tetrafluorethan | | 30,0 | | | 33,3 |
| Heptafluorpropan | 94,843 | | 98,843 | 98,35 | |

| Rezepturbeispiel Nr. | 18 | 19 | 20 | 21 | 22 | 23 |
|---|---|---|---|---|---|---|
| Salbutamol-Base | 0,142 | | | | | |
| Salbutamolsulfat | | 0,142 | | | | |
| Dinatriumcromoglicat | | | | 1,587 | | |
| Ipratropiumbromid | | | 0,029 | | | |
| Beclomethasondipropionat | | | | | 0,367 | |
| Budesonid | | | | | | 0,312 |
| MYVACET 9-45 | 0,021 | 0,021 | 0,001 | 0,039 | 0,001 | 0,039 |
| TWEEN 60 | 0,142 | 0,142 | | 0,158 | | 1,171 |
| Ethanol | 5,642 | 5,642 | 0,735 | 11,00 | 0,183 | 11,50 |
| n-Butan | | | | | | |
| Heptafluorpropan | 94,053 | 94,053 | 99,235 | 87,216 | 99,449 | 86,978 |

Mit den obigen Rezepturbeispielen lassen sich leicht redispergierbare Suspensions-Dosieaerosole herstellen. Für eine bessere Homogenisierung und zur Vermeidung von Adsorptionen ist die Zugabe von 0,5 - 20 %, insbesondere 0,5 - 12 %, Ethanol bevorzugt.

**Patentansprüche**

1.  Pharmazeutische Zusammensetzung zur Anwendung als Aerosol enthaltend:

8

a) ein verflüssigtes Treibgas oder Treibgasgemisch mit einem Dampfdruck von mehr als 1 bar und weniger als 6 bar (20°C) aus der Gruppe der unsubstituierten oder partiell bis vollständig fluorierten Kohlenwasserstoffe;

b) ein nicht-ionisches Tensid aus der Gruppe der monoacetylierten oder diacetylierten Monoglyceride;

c) einen pharmazeutischen Wirkstoff oder eine Wirkstoffkombination und gegebenenfalls

d) weitere übliche pharmazeutische Hilfsstoffe, welche sich für Aerosolformulierungen eignen.

2. Pharmazeutische Zusammensetzungen gemäss Anspruch 1 enthaltend:

a) ein Treibgas oder Treibgasgemisch aus der Gruppe Propan, n-Butan, Isobutan, Di-, Tri- oder Tetrafluorethan (134a) und Heptafluorpropan (227);

b) ein nicht-ionisches Tensid aus der Gruppe der monoacetylierten Monoglyceride;

c) einen pharmazeutischen Wirkstoff oder eine Wirkstoffkombination aus der Gruppe der Antiallergika, Betasympathomimetika, Anticholinergika, und Kortikoide und gegebenenfalls

d) weitere übliche pharmazeutische Hilfsstoffe, welche sich für Aerosolformulierungen eignen.

3. Pharmazeutische Zusammensetzungen gemäss Anspruch 1 enthaltend:

a) ein Treibgas oder Treibgasgemisch aus der Gruppe Propan, n-Butan, Isobutan, Tetrafluorethan (134a) und Heptafluorpropan (227);

b) ein nicht-ionisches Tensid aus der Gruppe der monoacetylierten Monoglyceride;

c) einen pharmazeutischen Wirkstoff oder eine Wirkstoffkombination aus der Gruppe Dinatriumcromoglicat, Salbutamol, Salmeterol, Formoterol, Oxitropiumbromid, Ipratropiumbromid, Budesonid, Flunisolid, Beclometason und Triamcinolon und gegebenenfalls

d) weitere übliche pharmazeutische Hilfsstoffe, welche sich für Aerosolformulierungen eignen.

4. Pharmazeutische Zusammensetzungen gemäss Anspruch 1 enthaltend:

a) das Treibgas Heptafluorpropan (227);

b) ein nicht-ionisches Tensid aus der Gruppe der monoacetylierten Monoglyceride;

c) einen pharmazeutischen Wirkstoff oder eine Wirkstoffkombination aus der Gruppe Dinatriumcromoglicat, Salbutamol, Salmeterol, Formoterol, Oxitropiumbromid, Ipratropiumbromid, Budesonid, Flunisolid, Beclometason und Triamcinolon und gegebenenfalls

d) weitere übliche pharmazeutische Hilfsstoffe, welche sich für Aerosolformulierungen eignen.

5. Verfahren zur Herstellung der pharmazeutischen Zusammensetzung gemäss Anspruch 1, dadurch gekennzeichnet, dass man in einem druckdichten Behältnis den pharmazeutischen Wirkstoff oder die Wirkstoffkombination c) vorlegt und in beliebiger Reihenfolge der Massnahmen das nicht-ionische Tensid b), gegebenenfalls die üblichen pharmazeutischen Hilfsstoffe d), und dazu das Treibgas oder Treibgasgemisch a) einleitet, homogenisiert und die homogene Mischung in für Aerosolformulierungen geeignete Dosierbehälter abfüllt.

6. Pharmazeutische Zusammensetzung gemäss Anspruch 1 zur Anwendung als Aerosolformulierung in einem therapeutischen Verfahren am menschlichen oder tierischen Körper.

7. Pharmazeutische Zusammensetzung gemäss Anspruch 1 zur Anwendung als Aerosolformulierung in der Inhalationstherapie zur Behandlung von Erkrankungen der Luftwege.

8. Verwendung eines nicht-ionischen Tensids aus der Gruppe der monoacetylierten oder diacetylierten Monoglyceride zur Herstellung einer pharmazeutischen Zusammensetzung, welche als Aerosolformulierung anwendbar ist.

**Patentansprüche für folgende Vertragsstaaten : ES, GR**

1. Verfahren zur Herstellung einer pharmazeutischen Zusammensetzung zur Anwendung als Aerosol enthaltend:

a) ein verflüssigtes Treibgas oder Treibgasgemisch mit einem Dampfdruck von mehr als 1 bar und weniger als 6 bar (20°C) aus der Gruppe der unsubstituierten oder partiell bis vollständig fluorierten Kohlenwasserstoffe;

b) ein nicht-ionisches Tensid aus der Gruppe der monoacetylierten oder diacetylierten Monoglyceride;

c) einen pharmazeutischen Wirkstoff oder eine Wirkstoffkombination und gegebenenfalls

d) weitere übliche pharmazeutische Hilfsstoffe, welche sich für Aerosolformulierungen eignen, dadurch gekennzeichnet, dass man in einem druckdichten Behältnis den pharmazeutischen Wirkstoff oder die Wirkstoffkombination c) vorlegt und in beliebiger Reihenfolge der Massnahmen das nicht-ionische Tensid b), gegebenenfalls die üblichen pharmazeutischen Hilfsstoffe d), und dazu das Treibgas oder Treibgasgemisch a) einleitet, homogenisiert und die homogene Mischung in für Aerosolformulierungen geeignete Dosierbehälter abfüllt.

2.   Verfahren zur Herstellung einer pharmazeutischen Zusammensetzung gemäss Anspruch 1 enthaltend:
a) ein Treibgas oder Treibgasgemisch aus der Gruppe Propan, n-Butan, Isobutan, Di-, Tri- oder Tetrafluorethan (134a) und Heptafluorpropan (227);
b) ein nicht-ionisches Tensid aus der Gruppe der monoacetylierten Monoglyceride;
c) einen pharmazeutischen Wirkstoff oder eine Wirkstoffkombination aus der Gruppe der Antiallergika, Betasympathomimetika, Anticholinergika, und Kortikoide und gegebenenfalls
d) weitere übliche pharmazeutische Hilfsstoffe, welche sich für Aerosolformulierungen eignen, dadurch gekennzeichnet, dass man die in Anspruch 1 bezeichneten Massnahmen durchführt.

3.   Verfahren zur Herstellung einer pharmazeutischen Zusammensetzung gemäss Anspruch 1 enthaltend:
a) ein Treibgas oder Treibgasgemisch aus der Gruppe Propan, n-Butan, Isobutan, Tetrafluorethan (134a) und Heptafluorpropan (227);
b) ein nicht-ionisches Tensid aus der Gruppe der monoacetylierten Monoglyceride;
c) einen pharmazeutischen Wirkstoff oder eine Wirkstoffkombination aus der Gruppe Dinatriumcromoglicat, Salbutamol, Salmeterol, Formoterol, Oxitropiumbromid, Ipratropiumbromid, Budesonid, Flunisolid, Beclometason und Triamcinolon und gegebenenfalls
d) weitere übliche pharmazeutische Hilfsstoffe, welche sich für Aerosolformulierungen eignen, dadurch gekennzeichnet, dass man die in Anspruch 1 bezeichneten Massnahmen durchführt.

4.   Verfahren zur Herstellung einer pharmazeutischen Zusammensetzung gemäss Anspruch 1 enthaltend:
a) das Treibgas Heptafluorpropan (227);
b) ein nicht-ionisches Tensid aus der Gruppe der monoacetylierten Monoglyceride;
c) einen pharmazeutischen Wirkstoff oder eine Wirkstoffkombination aus der Gruppe Dinatriumcromoglicat, Salbutamol, Salmeterol, Formoterol, Oxitropiumbromid, Ipratropiumbromid, Budesonid, Flunisolid, Beclometason und Triamcinolon und gegebenenfalls
d) weitere übliche pharmazeutische Hilfsstoffe, welche sich für Aerosolformulierungen eignen, dadurch gekennzeichnet, dass man die in Anspruch 1 bezeichneten Massnahmen durchführt